# EUROPEAN PATENT APPLICATION

(11) **EP 4 023 182 A1**
(43) Date of publication of application: **06.07.2022**
(21) Application number: 20218002.2
(22) Date of filing: 31.12.2020
(51) Int. Cl.: A61B 34/20

(54) **GENERATING A MAPPING FUNCTION FOR TRACKING A POSITION OF AN ELECTRODE**

(71) Applicant: Koninklijke Philips N.V., 5656 AG Eindhoven (NL)
(72) Inventor: WILDEBOER, Rogier Rudolf, 5656 AE Eindhoven (NL); HENDRIKS, Bernardus Hendrikus Wilhelmus, 5656 AE Eindhoven (NL); JANSSEN, Rik Jozef Martinus, 5656 AE Eindhoven (NL); SCHÄFER, Dirk, 5656 AE Eindhoven (NL)
(74) Representative: Philips Intellectual Property & Standards

(57) **Abstract**

A mechanism for generating a mapping function for mapping measurements taken at an electrode within crossing electric fields to a position or positions within a multidimensional co-ordinate system. The values for coefficients of the mapping function are generated using a first machine-learning algorithm, which receives, as input, example measurements (or responses) of the electrode and provides, as output, values for the coefficients for the mapping function. There is proposed a method and processing system for carrying out this mechanism.

## Description

### FIELD OF THE INVENTION

The present invention relates to the field of electrode positional tracking, and in particular, for mapping functions that facilitate the tracking of electrodes within crossing electric fields.

### BACKGROUND OF THE INVENTION

The accurate tracking of the position of an electrode (or electrodes) within a subject is becoming increasingly important for performing accurate and safe invasive procedures on a subject.

In an electric-field based electrode tracking system, two or more crossing electrical fields are induced by an array of electrodes positioned on the outside of the subject ("external electrodes"), such as on the surface of the subject. These electric fields induce position dependent responses, such as a voltage response, in electrodes placed within the body (an "internal electrode"). The position of an internal electrode can thereby be tracked by monitoring the response of the electrode to these electric fields. In particular, an appropriate mapping function can be used to map an electrical response of an internal electrode to a position within a multidimensional (Euclidian/Cartesian) co-ordinate system.

One scenario in which tracking the position of an electrode is important is in ablation procedures, where correct positioning of an interventional device (which may mount such an electrode) is important to minimize unintentional damage to the subject, such as damage caused by performing ablation at an incorrect location.

Another scenario in which accurate tracking of the position of an electrode is important is in medical imaging, a key element in diagnosing and/or treating a subject. A dielectric imaging process comprises tracking and iteratively recording the position of one or more internal electrode(s), and (re)constructing an anatomical model of the internal anatomy of the subject based on the recorded positions. Construction of the anatomical model is possible because it can be assumed that the internal electrodes will only be located within cavities of the subject, thereby allowing the bounds of the cavity to be constructed.

One example of a suitable mechanism for constructing a model of the internal anatomy of the subject based on the response of internal electrodes to crossing electric fields induced by external electrodes is disclosed by the European Patent Application having the publication number EP 3568068 A1.

There is an ongoing desire to improve the mapping function used to convert an electrical response (to the crossing electric fields) of an internal electrode to a position in a multidimensional co-ordinate system. This would improve the accuracy of tracking the position of an electrode and/or the accuracy of any anatomical model generated by processing tracked positions.

### SUMMARY OF THE INVENTION

The invention is defined by the claims.

According to examples in accordance with an aspect of the invention, there is provided a computer-implemented method for generating a mapping function that maps a response of an electrode to crossing electric fields within a subject, to a position in a multidimensional co-ordinate space.

The computer-implemented method comprises: obtaining electrical responses of two or more electrodes mounted on an interventional device to the crossing electric fields induced within the subject; processing the obtained electrical responses using a first machine-learning algorithm to obtain one or more values for a respective one or more parameters of the mapping function; and defining the mapping function based on the obtained one or more values for the respective one or more parameters of the mapping function.

The present disclosure makes use of a machine-learning algorithm to determine parameters and/or characteristics of the mapping function used to transform a response (e.g. a voltage response) of an electrode to a position within a multidimensional co-ordinate space. In particular, the first machine-learning algorithm determines values for parameters (e.g. coefficients) of the mapping function.

Conventionally, a minimization function is used to determine the values for the parameters of the mapping function, e.g. so that positions predicted by the mapping function adhere to known characteristics of the electrodes (such as a distance or spacing between the two or more electrodes). Another approach could be to use an analytical solution to determine the values for the parameters, e.g. using linear regression. The present disclosure recognizes that a machine-learning algorithm could be used instead of pre-existing approaches to calculate values for the parameters of the mapping function. It is herein recognized that such an approach improves the speed of and/or computational processing power required for (accurately) calculating the values for the parameters/coefficients of the mapping function.

The proposed approach thereby provides a mechanism for improved generating of a mapping function, by the use of a machine-learning algorithm to define the values for parameters (e.g. weights or coefficients for variables of the mapping function). This provides an improved approach for generating the mapping function.

In some examples, an initial mapping function may be obtained, e.g. having template, blank or predetermined values for certain coefficients. The first-machine learning algorithm may define values for weight/coefficients (or other parameters) of the generic mapping function, to effectively tune the generic mapping function for the specific use-case scenario.

The first machine-learning algorithm may be specific to the type of the interventional device and/or other properties of the interventional device. This embodiment recognizes that the properties of the interventional device have an impact upon how the mapping function should be generated, as the relationship between different positions predicted by a mapping function will differ depending upon the interventional device. In particular examples, the first machine-learning algorithm is dependent on the geometrical, electrical and/or mechanical properties of the interventional device, and in particular, the spacing between electrodes mounted on the interventional device.

The method may further comprise a step of obtaining an initial mapping function, and the step of defining the mapping function may comprise defining one or more parameters of the initial mapping function based on the obtained one or more values.

In some examples, the initial mapping function is specific to the type of the interventional device and/or the obtained electrical responses. Preferably, the first machine-learning is dependent upon the initial mapping function.

The method may further comprise a step of obtaining the first machine-learning algorithm by processing the obtained electrical responses using a second machine-learning algorithm to identify one of a plurality of potential first machine-learning algorithms to use as the first-machine learning algorithm.

Thus, a second (different) machine-learning algorithm can be used to predict which first machine-learning algorithm should be used to generate the values for the parameters of the mapping function (e.g. values for the weights/coefficients of a generic or base mapping function).

This approach facilitates a simple and automated approach for selecting an appropriate first machine-learning algorithm for use in generating the mapping function. In turn, this provides a more accurate mechanism for generating the values for the generated mapping function.

Of course, other approaches for obtaining the first machine-learning algorithm could be employed, e.g. using a single first machine-learning algorithm, using a look-up table (e.g. to correlate information about the interventional device to a particular first machine-learning algorithm), using a use-set decision tree or the like.

In some examples, the step of obtaining the first machine-learning algorithm comprises: processing the obtained electrical responses using the second machine-learning algorithm to identify a type of the interventional device; and identifying which potential first machine-learning algorithm to use as the first machine-learning algorithm based on the identified type of the interventional device.

This embodiment recognizes that different types of interventional device should be treated differently in the generating of the values for the mapping function. This embodiment also appreciates that a type of an interventional device can be predicted based on the electrical responses generated by the interventional device (e.g. due to a difference in a relationship between electrical responses between different interventional devices and the like).

An alternative approach could be to use a look-up table or the like (e.g. to map electrical responses to a type of interventional device). Yet another approach could be to receive a user input indicating the type of interventional device, and identify the potential first machine-learning algorithm to use as the first machine-learning algorithm based on the user-identified type of interventional device.

Optionally, the step of processing the obtained electrical responses comprises: normalizing the obtained electrical responses; and processing the normalized obtained electrical responses using the first machine-learning algorithm to obtain the one or more values for the respective one or more parameters of the mapping function. Normalizing the obtained electrical responses means that they are more readily usable as network input, and in particular, can prevent a "blowing up" of a machine-learning algorithm.

Other pre-processing steps may otherwise/additionally be performed upon the obtained electrical responses, e.g. centering/centralization, noise removal, movement compensation, truncation, filtering, etc. These approaches may make the electrical responses more readily interpreted by a machine-learning algorithm, and improve the accuracy of the output of the machine-learning algorithm.

The step of obtaining electrical responses may comprise obtaining at least some of the electrical responses from two or more electrodes mounted on an interventional device positioned within the subject. In other examples, the electrical responses are obtained from a memory that stores the electrical responses of two or more electrodes mounted on the interventional device. Of course, a combination of both of these approaches could be used (e.g. the electrical responses could be obtained from the interventional device and/or a memory).

Preferably, the first machine-learning algorithm is trained using computer-generated synthetic electrical responses. Of course, it is not essential that the first machine-learning algorithm be trained using exclusively synthetic electrical responses (e.g. "real" or input electrical responses could be used in addition to or instead of the synthetic electrical responses).

The electrical responses may be electrical responses of the one or more electrodes within a predetermined part of an anatomical cycle of the subject. An anatomical cycle is any predictable cyclic motion or movement of the subject, such as a cardiac cycle or a respiratory cycle. Other suitable predictable anatomical cycles of a subject will be readily apparent to the skilled person.

This embodiment recognizes that the most appropriate mapping function (for accurate prediction of position) may change during an anatomical cycle of the subject, e.g. due to changes in dielectric properties of a subject during the anatomical cycle, which result in changes to the electrical response of an electrode located at a same position in the subject.

By effectively gating the obtained electrical responses (i.e. so that they correspond only to a predetermined part of the anatomical cycle), the mapping function can be made specific to a particular part of the anatomical cycle for improved consistency and accuracy in tracking a position of an electrode within the crossing electrical fields.

In some examples, the electrical responses are filtered and/or modified to compensate for the behavioral response of (i.e. the expected change in) the electrical responses during the course of the anatomical cycle, e.g. through the use of motion compensation.

In particular examples, different mapping functions can be produced or generated for different parts of the anatomical cycle. Thus, the described method may be repeated using different electrical responses for different parts of the anatomical cycle. This approach facilitates more continuous tracking of the position of an electrode.

In some embodiments, the first machine-learning algorithm is dependent upon the predetermined part of the anatomical cycle of the subject.

The first machine-learning algorithm may comprise a recursive neural network, a convolutional neural network and/or a deep unfolding network. Other suitable forms of a machine-learning algorithm will be apparent to the skilled person.

There is also proposed a computer-implemented method of identifying the position, in a multidimensional co-ordinate space, of an electrode within a subject.

The computer-implemented method comprises: obtaining a mapping function, such as those generated using a method herein described; obtaining the electrical response of an electrode to crossing electric fields within the subject; and determining a position, in a multidimensional co-ordinate space, of the electrode by processing the electrical response of the electrode using the mapping function. Other mapping functions (e.g. generated using conventional approaches) may be used in other aspects of the inventive concept.

The method may further comprise obtaining indication data responsive to a change in one or more dielectric properties of elements within the crossing electric field and/or a property of the interventional device and/or electrodes; and in response to the indication data indicating a change, regenerating a mapping function, e.g. by performing a herein described method.

This embodiment recognizes that changes to the dielectric properties of elements within the crossing electric fields (e.g. changes in anatomical structure due to ablation or the like) or the interventional device (e.g. an inflation of a cryoballoon) affect the accuracy of a mapping function. By regenerating the mapping function responsive to such changes, an accuracy of the mapping function can be maintained/preserved.

There is also proposed a computer-implemented method of generating an anatomical model of an anatomical cavity, the computer-implemented method comprising: iteratively identifying the position of one or more electrodes within an anatomical cavity by performing a herein described method; and generating an anatomical model of the anatomical cavity by processing the identified positions of the one or more electrodes. Mechanisms for generating an anatomical model based on the recorded positions of an electrode (or electrodes) within an anatomical cavity are well known to the skilled person.

There is also proposed a computer program product comprising computer program code means which, when executed on a computing device having a processing system, cause the processing system to perform all of the steps of any herein described method.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

There is also proposed a processing system for generating a mapping function that maps a response of an electrode to crossing electric fields within a subject, to a position in a multidimensional co-ordinate space.

The processing system is configured to: obtain, at an input interface, electrical responses of two or more electrodes mounted on an interventional device to the crossing electric fields induced within the subject; process the obtained electrical responses using a first machine-learning algorithm to obtain one or more values for a respective one or more parameters of the mapping function; and define the mapping function based on the obtained one or more values for the respective one or more parameters of the mapping function.

The skilled person would be readily capable of adapting the processing system to perform any herein described method, and vice versa.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates positions for a set of external electrodes positioned on a subj ect;
Figure 2 illustrates a co-ordinate system for defining a direction of electric fields generated by the external electrodes;
Figure 3 illustrates a processing system;
Figure 4 illustrates an approach for reconstructing an anatomical model from a point cloud;
Figure 5 illustrates a mapping function;
Figure 6 is a flowchart illustrating a method;
Figure 7 is a flowchart illustrating another method;
Figure 8 is a block diagram illustrating a processing system; and
Figure 9 illustrates a processing arrangement comprising a processing system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating a mapping function for mapping measurements taken at an electrode within crossing electric fields to a position or positions within a multidimensional co-ordinate system. The values for coefficients of the mapping function are generated using a first machine-learning algorithm, which receives, as input, example measurements (or responses) of the electrode and provides, as output, values for the coefficients for the mapping function. There is proposed a method and processing system for carrying out this mechanism.

Embodiments are based on the realization that the process for generating a mapping function is a complex task, which can be improved through the use of machine-learning algorithms. Moreover, it is recognized that a process of generating values for coefficients of the mapping function can be achieved using a machine-learning algorithm, rather than conventional minimization/linear regression approaches. This facilitates more flexible generation of mapping functions.

Concepts of the invention may be employed in any circumstances in which it would be beneficial to map a response within crossing electric fields induced in a subject to a position in space, such as for tracking an electrode during a surgical/interventional procedure and/or for performing imaging of an anatomical cavity.

For the purposes of contextual understanding, the principle and purpose of inducing electric fields within a subject (i.e. an individual, such as a person or animal) will be hereafter described.

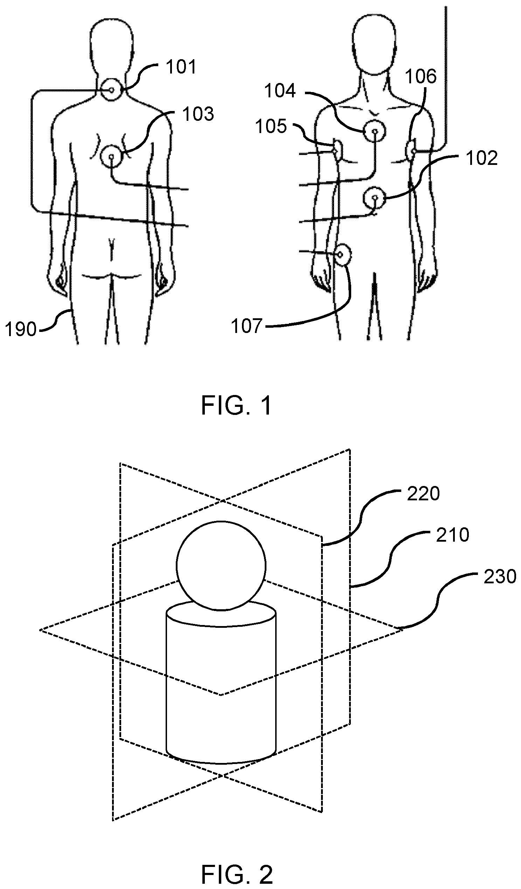

Figure 1 illustrates positions for a set of external electrodes positioned on a subject 190. External electrodes are electrodes that are positioned externally to a subject, and are contrasted with internal electrodes that are positionable within the subject.

The illustrated set of external electrodes comprises a first external electrode 101, a second external electrode 102, a third external electrode 103, a fourth external electrode 104, a fifth external electrode 105 and a sixth external electrode 106. The illustrated set further comprises a reference electrode 107, which can act as a "ground" for defining a base/background level of electric field activity in the subject.

An electric signal provided to each external electrode is controlled to thereby define crossing electric fields between the electrodes. In particular, the first 101 and second 102 external electrodes form a first pair of external electrodes ("external electrode pair"), and signals provided to the first external electrode pair can be controlled to induce a first electric field therebetween. The third 103 and fourth 104 external electrodes form a second pair of external electrodes, and signals provided to the second external electrode pair can be controlled to induce a second electric field therebetween. The fifth 105 and sixth 106 external electrodes form a third pair of external electrodes, and signals provided to the third external electrode pair can be controlled to induce a third electric field therebetween.

Electric signals provided to each external electrode, and in particular to each pair of electrodes, can control the frequency and/or magnitude/strength of the crossing electric fields. The electric signals supplied to the electrodes may be controlled by an electric field generator (not illustrated in Figure 1).

The crossing electric fields are usable to track or identify a location of electrodes positioned within the (overlap of the) crossing electric fields. In particular, a response of an electrode, e.g. a voltage response, to each electric field will change as a position within the electric field changes (e.g. a distance from a source/sink of the electric field changes). The response of an electrode to the crossing electric fields can be mapped or associated with a particular position within a multidimensional co-ordinate system using a mapping function.

In other words, an electrical response (which can be alternatively labelled a "measurement") of the internal electrode to the crossing electric fields can be processed to determine a relative position of the internal electrode (e.g. and therefore of any interventional device comprising the internal electrode) within an anatomical cavity of the subject.

In particular, there may be provided a mapping function mapping an electrical response of an electrode to a position within a multidimensional co-ordinate space (i.e. to a position in space, namely a Euclidian/Cartesian co-ordinate space).

The mapping function is required because the electric fields are non-linear, meaning that a position defined using a response alone (i.e. without mapping) does not necessarily relate to a position in actual space. The coefficients (values of parameters) of the mapping function represent scaling factors and/or local distortions to map a position in the electric field space (or V-space, if voltage responses are used) to a predicted position in a "true" space (or R-space).

As a very simple example, the mapping function may map an electrical response Vx to a position Rx. One example of a mapping function takes the form of Rx = a^{∗}Vx+b. The mapping function thereby contains coefficients or parameters a and b, for which values need to be acquired. Other, more complex mapping functions, may be used instead.

Each electric field may be controlled to have a particular/different frequency. This facilitates determination of the relative position of an electrode with respect to each electric field, by assessing the response of the electrode to the particular frequency of the electric field. This information can be used to effectively identify or predict the position of the electrode with respect to a co-ordinate system defined by the electric fields.

One of the external electrode pairs may be omitted, e.g. if only two crossing electric fields are desired (e.g. for performing a 2-dimensional tracking process).

Figure 2 schematically illustrates a co-ordinate system for defining a direction of electric fields generated by the external electrodes. The co-ordinate system defines three cardinal planes 210, 220, 230. The crossing electric fields may, for instance, be optimally positioned when the direction of each electric field is parallel to a respective cardinal place 210, 220, 230.

The intent of the crossing electric fields, e.g. such as those generated using the set illustrated by Figure 1, is to facilitate identification of the position of an electrode (or group of electrodes) with respect to a co-ordinate system, such as that illustrated by Figure 2.

A more complete example of how to track the position of an internal electrode with respect to crossing electric fields (i.e. within the subject), and optionally how to further exploit this information, e.g. for the construction of an anatomical model of an anatomical cavity of the subject, is hereafter described for the purposes of contextual understanding.

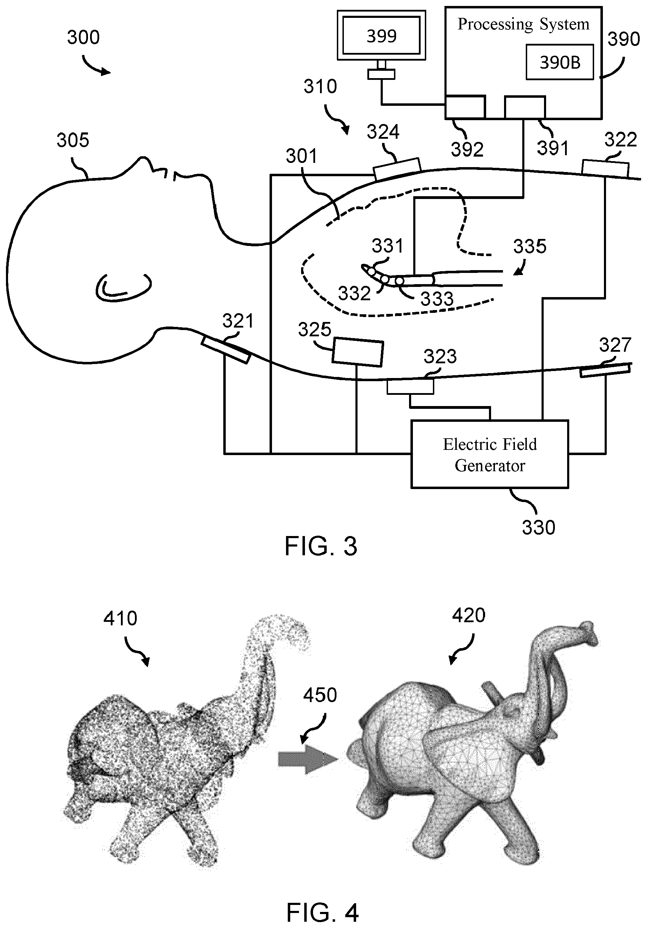

Figure 3 conceptually illustrates a processing arrangement 300 for generating a mapping function for predicting the position of an electrode within an anatomical cavity 301 of a subject 305 (e.g. blood vessels, gastrointestinal tract, the cardiac system and so on). The processing arrangement 300 may further generate an anatomical model of an anatomical cavity within a subject.

In particular examples, the processing arrangement 300 may be configured to generate an anatomical model (of an anatomical cavity, such as a blood vessel and/or chamber) using a dielectric imaging process.

The processing arrangement 300 comprises an exemplary electric field generating arrangement 310 and a processing system 390.

The processing system 390 is configured to generate a mapping function for mapping electrical responses of electrodes to a position within a multidimensional co-ordinate space (i.e. to a position in a Euclidian/Cartesian co-ordinate space). Thus, the processing system 390 acts as a mapping function generator.

The processing system 390 may also contain a dielectric imaging processor 390B, although this may be alternatively positioned externally to the processing system 390.

The electric field generating arrangement 310 comprises a set of external electrodes 321, 322, 323, 324, 325, 327 positioned externally with respect to the subject 305 (e.g. as electrode patches provided on a skin of the subject). The set 310 of external electrodes may comprise a plurality of electrode pairs angled with respect to one another (e.g. orthogonal to one another), so that any electric fields generated by the electrode pairs are angled with respect to one another. These electrode pairs may comprise a first electrode pair (formed of a first 321 and second 322 external electrode), a second electrode pair (formed of a third 323 and fourth 324 external electrode) and a third electrode pair (formed of a fifth 325 and sixth (not visible) external electrode). One of more of these electrode pairs may be omitted. The set of external electrodes may also comprise a reference electrode 327. The external electrodes are placable in a similar manner to the set of external electrodes illustrated in Figure. 1.

The electric field generating arrangement 310 also comprises an electric field generator 330, which is adapted to control (characteristics of) an electric signal (e.g. voltage and/or current) supplied to each external electrode. In some examples, this may form part of the processing system 390.

The electric field generating arrangement 310 is configured to generate multiple (here: three) crossing (intra-body) electrical fields using the external electrodes. This is performed using appropriate control of the electric signals provided to each external electrode.

In particular, each electrode pair may be appropriately controlled to induce an electric field between each electric pair. Thus, where there are three electrode pairs, three electric fields may be generated. Preferably, the frequency of each generated electric field is controlled to be different, to facilitate increased ease in identifying a relative location of an electrode positioning within the crossing electric fields.

The generated electric fields can be used to define or establish a relative location of an (internal) electrode positioned within the crossing electric fields. In particular, as previously explained, the (electrical) response of an internal electrode to the electric fields changes as the relative position of the internal electrode moves about the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the relative position of the internal electrode using a processing system 390 that monitors a response of any internal electrodes to the crossing electric fields, e.g. by mapping the (electrical) response of an electrode to a relative position within the subject or using a suitable mapping/transfer function, e.g. the "V2R function" described below, to determine the relative position(s) of the electrodes.

By way of further explanation, for the purposes of improved conceptual understanding, if the crossing electric fields are controlled to have a different frequency with respect to one another, then the response of the internal electrode to each frequency could be used to determine a relative distance between from each source/sink of the corresponding electric field. This principle can be used to effectively triangulate the relative position of the internal electrode within the crossing electric fields.

For instance, if there are three external electrode pairs, positioned to emit electric fields (E₁, E₂, E₃) of different frequencies that are angled (e.g. near-orthogonal) with respect to one another, a voltage response (V₁, V₂, V₃) of an internal electrode (identifying a voltage (e.g. between the electrode and the reference electrode or between the electrode and the electrode generating the electric field) at each of these three frequencies) will differ depending upon position within the anatomical cavity.

The skilled person will appreciate that determining the position of internal electrodes 331, 332, 333 also facilitates determining the position, orientation and/or angle of an interventional device 325 that mounts the electrodes. In particular, a positional relationship of the electrodes on the interventional device may be known/predetermined, and used to derive an orientation of the interventional device (e.g. define an axis along which the interventional device lies).

Other forms of response for an internal electrode (e.g. an impedance response or a capacitive response, e.g. indicating change in impedance/capacitance between the internal electrode and an external electrode) will be apparent to the skilled person.

The electric field generator 330 may be configured to control the electric fields, generated using the set of external electrodes, to operate in the frequency range of 30-100 kHz. This frequency range is particularly useful for ensuring penetration of a subject, whilst providing a frequency range that attenuates in human tissue without significant damage/injury to the tissue. The reference electrode serves as an electric reference for all measurements taken by electrodes, e.g. as a reference for the response of the internal electrodes.

The response of two or more internal electrodes mounted upon a single interventional device can be used to construct (and update) the mapping function used to map a response of the internal electrode to a predicted position within the anatomical cavity (e.g. within some Euclidian/Cartesian space).

A conventional process for generating or constructing a mapping function ("V2R function") using a processing system is hereafter described, for improved contextual understanding. The present disclosure relates to a new/different process for generating this mapping function.

As previously mentioned, the processing system makes use of a plurality of internal electrodes 331, 332, 333 positioned and moved within the anatomical cavity (e.g. electrodes positioned on an interventional device 335 to be inserted into the anatomical cavity). A spatial relationship (e.g. a distance) between each of the internal electrodes may be predetermined and/or known.

The mapping function generator 390A is configured to receive (and optionally provide) signals (e.g. at an input interface 391) from/to the internal electrodes 331, 332, 333 to determine a response of the internal electrodes to the crossing electric fields.

The response of the internal electrodes 331, 332, 333 (e.g. to externally applied electric fields by the external electrodes) is then iteratively recorded for different positions and/or orientations of the interventional device 335, i.e. to obtain a "measurement" from each internal electrode as the interventional device is moved within the cavity 301. The processing system can then repeatedly define/update and apply a mapping function or transfer function ("V2R function") that transforms each recorded response to Euclidian/Cartesian coordinates (R-space), whilst ensuring known properties and/or spatial relationships of the internal electrodes and/or interventional device 335 (e.g. electrode spacing and electrical weight length) as well as a set of other constraints are maintained. For instance, the mapping/transfer function can effectively learn and linearize the distorted relation between the measured responses and the actual position in three dimensions by taking the known inter-electrode distances as a reference. By way of example only, if an internal electrode has a response matching a previously measured response then the relative distance to the responses measured by the other internal electrodes can be determined and used to improve the mapping/transfer function.

In other words, the mapping/transfer function effectively determines or predicts a relative/predicted location of each electrode in a Euclidian and/or multidimensional space or co-ordinate system ("R-space"). In this way, an R-space cloud of points (known Euclidian/Cartesian co-ordinates) can be built up and updated as the internal electrodes are moved within the anatomical cavity. This R-space cloud of points is then iteratively analyzed in order to iteratively update the mapping function to adhere to the known properties of the internal electrodes and/or interventional device, such as a spatial relationship of the internal electrodes.

Other descriptions and/or embodiments of the process for generating the mapping function are disclosed by the European Patent Applications EP 0775466 A2, EP 3568068 A1 and EP 3607879 A1.

The predicted positions of an internal electrode or internal electrodes may also be used to construct an anatomical model of an anatomical cavity (i.e. a cavity in which the interventional device is able to move). This process is called a dielectric imaging process, and may be performed by the dielectric imaging processor 390B.

Broadly, the dielectric imaging processor 390B may build an anatomical model of an anatomical cavity 301 (e.g. a chamber, vessel or void) within a subject by processing an R-space cloud of points. In particular, using an updated R-space cloud of points, such as the R-space cloud of points produced during the construction of the mapping function, a reconstruction algorithm generates an anatomical model of the (investigated part of the) anatomical cavity. The anatomical model may, for example, be a 3D surface that depicts or models the (known bounds of) the anatomical cavity.

The process of reconstructing an anatomical model from a point cloud is conceptually illustrated in Figure 4, which demonstrates a process 450 in which a cloud of R-space points 410 ("point cloud") is transformed into an anatomical model 420. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

In this way, the "global field" measurements (i.e. the effect of the electric fields generated by external electrodes on the internal electrodes) can be used to generate a rough anatomical model of the anatomical cavity.

Referring back to Figure 3, more precise identification of the bounds and features of the anatomical model can be performed by monitoring the response of the internal electrodes 331, 332, 333 to local electric fields (e.g. fields generated by other internal electrodes). Thus, in some embodiments, the dielectric imaging processor 390B may also control the internal electrodes 331, 332, 333 to generate an electric field (which can be detected by other internal electrodes). The response of the internal electrodes to local electric fields can be labelled "local field measurements", as compared to the response of the internal electrodes to externally induced electric fields which can be labelled "global field measurements".

For instance, changes in a local electrical field (being an electric field induced between two internal electrodes 331, 332, 333) can indicate the presence or absence of tissue between or near the two internal electrodes. A response of an internal electrode to a local electric field can therefore be used to identify the presence or absence of tissue, to thereby facilitate tuning or updating of the anatomical model.

In some examples, additional processing of global field measurements (i.e. the responses of the internal electrodes to electric fields induced by the external electrodes) can be performed to improve the precision of the bounds and features of the anatomical model.

For example, regions with inherently marked steep gradients in the electrical field responses can be identified. It is recognized that such regions indicate the bounds of the anatomical cavity and/or other information, e.g. drainage of vessels into or out of a cardiac chamber as well as the valves of a cardiac chamber. These features can thereby be uniquely identified by the system and imaged even without physically visiting them with the interventional device 335.

The combined global and local field measurements enable sophisticated detection and effective handling of inconsistencies and outliers, level of electrode shielding/coverage (e.g. by measuring location inter-correlation), pacing (saturation), as well as physiological drift. Drift can, for example, be detected using a moving window over time and corrected continuously whereby the internal electrode location remains accurate throughout the whole procedure making the system resilient to drift.

The above-provided description of a dielectric imaging process is only an example, and the skilled person would be readily capable of modifying the described process appropriately.

The skilled person will appreciate that the derived mapping/transfer function ("V2R function") can also be used to track a location of one or more internal electrodes with respect to a constructed anatomical model. This could facilitate the generation and display of an anatomical model and an indicator of a current location/position of an interventional device 335 mounting internal electrodes 331, 332, 333 with respect to the anatomical model. Of course, a visual representation of any generated anatomical model and/or determined position may be generated and provided at an output display 399.

The processing system 390 comprises an input interface 391 for receiving signals responsive to the electrical response of the internal electrodes to the electric fields generated by the electric field generating arrangement. The processing system 390 may also comprise an output interface 392 for providing signals for controlling the output display 399.

The present disclosure relates to a new mechanism for generating the mapping function, to facilitate an improved and/or more accurate mapping function. An improved mapping function will, in turn, improve the accuracy of tracking the position of an interventional device and/or the accuracy of an anatomical model generated using data obtained using a mapping function.

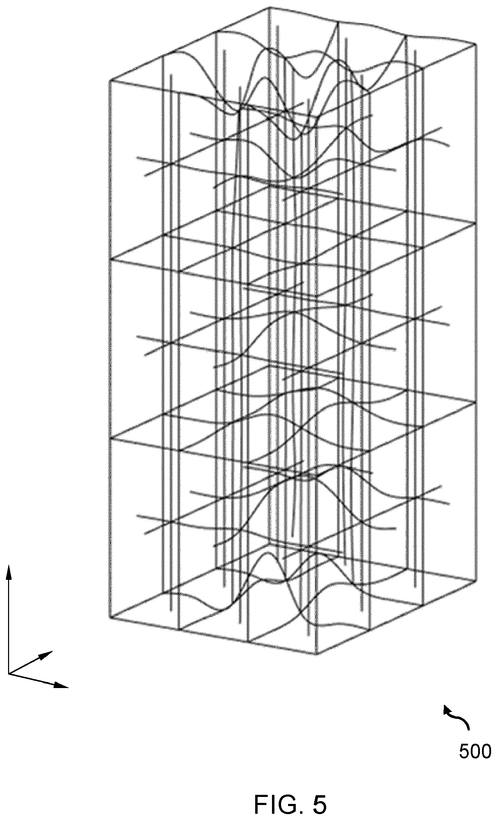

Figure 5 conceptually illustrates the problems facing determination of the parameters of the mapping function. In particular, Figure 5 illustrates an example mapping function 500 (i.e. a non-linear transformation) for mapping between an electrical response (e.g. a position in "V-space"), illustrated as the non-oblong grid and a position in a multidimensional co-ordinate space (e.g. a position in "R-space"), illustrated as the oblong grid.

This mapping function clearly demonstrates the field nonlinearities that occur in the crossing electrical fields, meaning that the electrical response is unable to directly represent a true position in a multidimensional co-ordinate space without transformation (by the mapping function). This emphasizes the non-triviality of the transformation, and the difficulty in obtaining accurate values for parameters/coefficients of the mapping function.

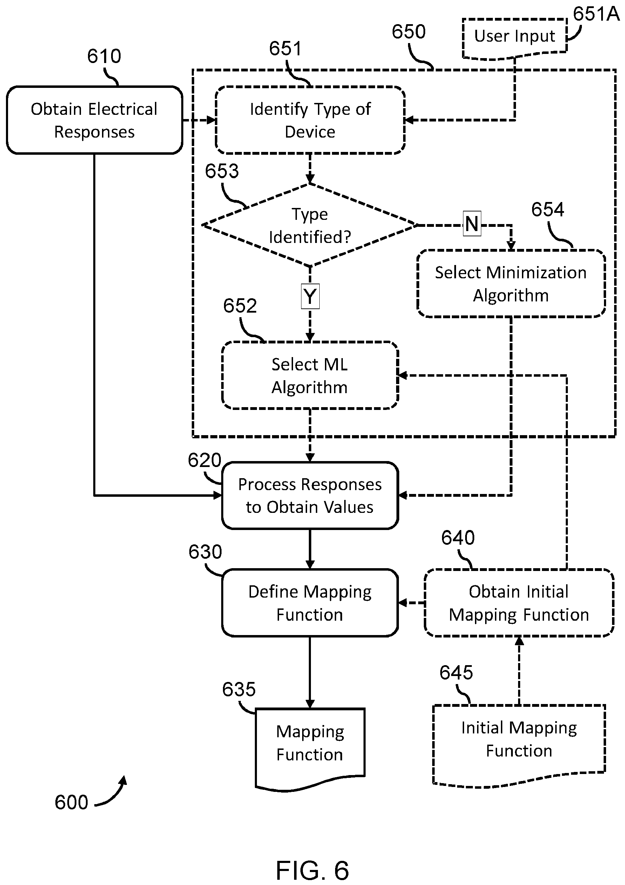

Figure 6 illustrates a computer-implemented method 600 for generating a mapping function 635 according to an embodiment of the invention.

The method 600 comprises a step 610 of obtaining electrical responses of two or more electrodes mounted on an interventional device to the crossing electric fields induced within the subject. The electrical responses may be obtained at an input interface (e.g. of a processing system, such as the processing system 390 of Figure 3).

The method 600 then moves to a step 620 of processing the obtained electrical responses to obtain respective value(s) for one or more parameters of the mapping function, i.e. identify coefficient values for the mapping function. Step 620 will, in some paths of the method 600, use a first machine-learning algorithm to obtain the one or more values.

The method 600 then moves to a step 630 of defining the mapping function 635 based on the obtained one or more values. In particular, the obtained value(s) may be inserted into the appropriate (i.e. corresponding) positions in the mapping function. The mapping function 630 may be output at an output interface (e.g. of a processing system).

Thus, the proposed method 600 makes use of a first-machine learning algorithm to determine coefficients for the mapping function, e.g. rather than a conventional minimization or analytical approach (such as linear regression). This provides a fast and efficient mechanism for generating the mapping function.

A machine-learning algorithm is any self-training algorithm that processes input data in order to produce or predict output data. Here, the input data comprises electrical responses of one or more electrodes to crossing electric fields, and the output data comprises values for parameters of a mapping function.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

For the present invention, a neural network such as a recursive neural network (RNN), a convolutional neural network (CNN) or a deep unfolding network (DUN) would be particularly advantageous.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries ("ground truth data"). An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example electrical responses of electrodes to crossing electric fields. The training output data entries correspond to associated values for parameters of the mapping function.

Although real-life data could be used to provide the training input data entries, it is also possible to make use of synthetic data. The use of synthetic data is particularly well suited for the first machine-learning algorithm(s), as a wide variety of mapping functions can be simulated and a large sets of possible input samples can be generated.

In some embodiments, data augmentation (of ground truth data and/or synthetic data) could be performed by adding, for example, noise to true or synthetic data.

In any case, it is preferable if the training dataset has sufficient variability in spatial coordinates and output variables, i.e. where a variability measure is above some predetermined threshold. In particular examples, the training dataset should be representative of a large percentage of the patient population in terms of, for example, heart (or other anatomical cavity of interest) size, anatomy, recorded electrical responses, and anatomical cycles.

Proper convergence during training may be realized by constraining the outputs of the neural network (or other first machine learning algorithm) such that there are no sudden jumps in the mapping function (e.g. so that the maximum difference between a change in input values and a corresponding change in output value for the mapping function is below some predetermined threshold). Additionally, constraints on the determined values for the mapping functions may be used to enforce a nondegenerate field distribution for the electric fields.

To improve the performance of the first machine-learning algorithm, the obtained response may be formatted to a specific (and therefore predictable) input data architecture. This approach helps make sure that the machine-learning algorithm captures the inter-electrode distances used for generating an accurate mapping function.

One feasible solution would be to format the obtained electrical responses into as a matrix (for inputting to the first machine-learning algorithm) of a predetermined size. For example, an MxDxN matrix may be defined, where M represents the number of electrodes on an interventional device, D represents the number of dimensions (i.e. the number of parts of the electrical response) and N represents a number of responses obtained.

As the total number of electrical responses obtained may be variable, zero-padding and/or a sliding window for all electrical responses could be used - i.e. to limit the number of obtained responses in the matrix to be equal to the value N.

To further improve the performance of the first machine-learning algorithm, additional pre-processing may be performed on the obtained responses. For example, whitening of the obtained electrical responses could be performed. Other pre-processing steps include normalization, centering/centralization, noise removal, movement compensation, truncation, filtering and so on. The skilled person would appreciate other pre-processing steps that would improve the performance of the machine-learning algorithm.

Some machine-learning algorithms are configured to provide a confidence measure indicating the confidence of a desired output (in this case: a confidence of the values for the parameters of the mapping function). Some embodiments of the invention may, if this confidence measure falls below a predetermined threshold, regenerate the mapping function (e.g. using a different first machine-learning algorithm, such as one trained on a narrower dataset) or generate the mapping function using a conventional, non-machine-learning algorithm reliant approach.

The method 600 may comprise a step 640 of obtaining an initial mapping function 645. This initial mapping function may contain template, blank or default values for certain parameters, which are replaced/set in step 630 based upon the values determined in step 620.

In some examples, there may be a plurality of potential first machine-learning algorithms that could be used, i.e. in step 620. The method 600 may comprise a step 650 of selecting an algorithm or approach for obtaining the values from the obtained electrical responses.

In some examples, a second machine-learning algorithm could be used to process the obtained electrical responses in a process of identifying which of the potential first machine-learning algorithms is to be used as the first machine-learning algorithm.

Preferably, the first machine-learning algorithm is selected based upon a type (or properties) of the interventional device. This embodiment recognizes that the geometrical, electrical and mechanical properties of the interventional device have an influence on how the parameters of the mapping function are generated. By taking the type of the interventional device into account, in selecting the first machine-learning algorithm, the accuracy of the subsequently generated mapping function is increased.

In some examples, step 650 comprises a step 651 of identifying a type or identity of the interventional device. Step 651 may, for instance, comprise receiving a user input 651A indicating a type of the interventional device. In other, preferred examples, step 651 comprises processing the obtained responses (from step 610) using a second machine-learning algorithm to identify the type of device.

The method may then perform a step 652 of selecting the first machine-learning algorithm to be used in step 620 (from a plurality of potential first machine-learning algorithms), based on the type of the interventional device. This may be performed, for instance, using a look-up table that maps a detected type of interventional device to a particular potential first machine-learning algorithm. In this way, the most appropriate first machine-learning algorithm for a particular type of interventional device may be selected.

In some examples, if step 651 is unable to identify the type of interventional device (e.g. as determined in a step 653), the method may select or instruct, in a step 654, a conventional algorithm or approach to be used (in step 620) for generating the value(s) for the parameter(s) of the mapping function.

Thus, in some paths of the method 600, the first machine-learning algorithm is not used, e.g. if an appropriate first machine-learning algorithm cannot be identified. In these circumstances, the method may revert to a conventional approach for generating the values for coefficients of the mapping function, e.g. using a minimization function.

In some examples, there may be a plurality of potential mapping functions that could be used, where different mapping functions have different parameters and/or combinations of inputs. Step 640 may be adapted to comprise selecting one of a plurality of potential mapping functions as the initial mapping function.

In these examples, if there is more than one potential first machine-learning algorithm, the selected first machine-learning algorithm may be dependent upon the selected mapping function. This ensures that the machine-learning algorithm is specifically selected to generate the outputs for a particular mapping function (i.e. the machine-learning algorithm is configured for the selected mapping function).

In other words, step 650 may be configured to select an algorithm for obtaining the values from the obtained electrical responses based on (i.e. responsive to) the selected initial mapping function. This may be, in some examples, implemented as a part of step 652.

In some examples, step 640 may specifically comprise selecting the initial mapping function based on the obtained electrical responses (e.g. by processing the obtained electrical responses using a (third) machine-learning algorithm or the like). In examples, step 640 may comprise selecting the initial mapping function based on the type of interventional device (e.g. if step 651 is performed). This may be performed, for instance, using a look-up table that maps a type of interventional device to a particular initial mapping function.

Temporal changes in a subject, e.g. by respiratory or cardiac motion - i.e. anatomical cycles, can significantly deteriorate the ability of neural networks or other machine-learning algorithms to generate appropriate parameters for the mapping function, as they generally assume a steady state. This problem can be mitigated by using an appropriate gating procedure when obtaining the prior to calculation of the mapping function, or by performing motion compensation on obtained electrical responses.

In other words, when performing a gating procedure, the obtained electrical responses (in step 610) may comprise only electrical responses within a predetermined part of an anatomical cycle of the subject, e.g. during an inhalation phase of a respiratory cycle or during an inflow or ejection phase of a cardiac cycle. Other suitable phases of anatomical cycles will be readily apparent to the skilled person.

In some examples, different mapping functions are generated for different phases or parts of an anatomical cycle. Put another way, the method 600 may be repeated for different specific parts of the anatomical cycle, with the obtained electrical responses being within the specific parts of the anatomical cycle.

This embodiment takes particular advantage of the speed of predicting parameters for values of the mapping function facilitated through use of the first machine-learning algorithm, making this approach more feasible to be performed in real-time, compared to conventional approaches.

In some examples, if there are a plurality of potential first machine-learning algorithms, the selected first machine-learning algorithm may be dependent upon the part of the anatomical cycle with which the obtained electrical responses are associated. This embodiment can further improve the specificity of the machine-learning algorithm. This may be incorporated into step 652.

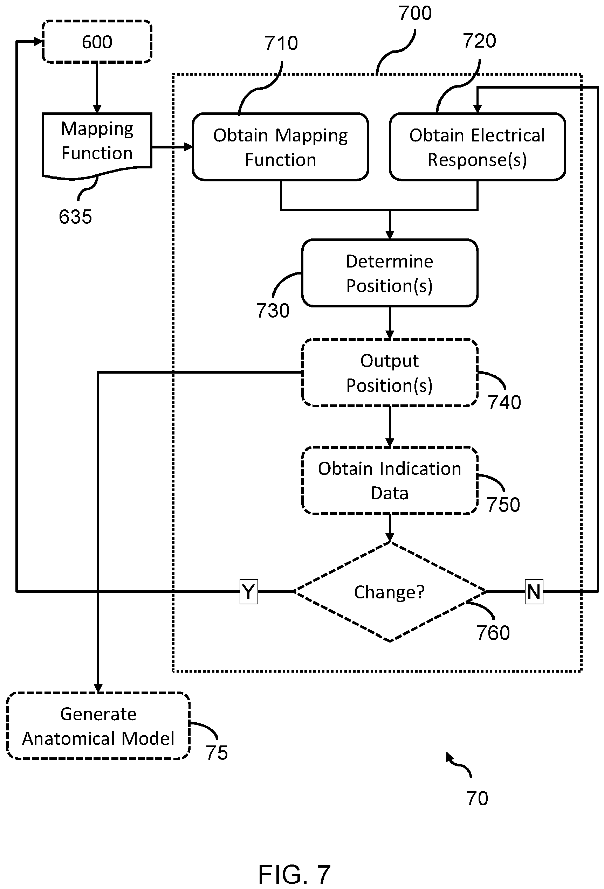

Figure 7 illustrates a computer-implemented method 700 according to another embodiment of the invention. The method 700 is configured for identifying the position, in a multidimensional co-ordinate space, of an electrode (or electrodes) within a subject.

The method 700 comprises performing a step of obtaining 710 a mapping function. This process may be performed, for instance, by obtaining the mapping function 635 generated by the method 600 previously described with reference to Figure 6.

The method 700 also comprises a step 720 of obtaining the electrical response(s) of an electrode to crossing electric fields within the subject. The obtained electrical response(s) should have the same (data) format as the electrical responses for which the mapping function 635 is configured to map to a position within the multidimensional space.

The method 700 also comprises a step 730 of processing the obtained electric response(s) using the mapping function, to thereby determine the position of the electrode(s) within the multidimensional space.

Thus, a mapping function is used to identify the position of the electrode. It has previously been explained how this information can be used to identify the location and/or orientation of an interventional device upon which the electrode(s) is/are mounted.

The identified position(s) may be output in a step 740. For instance, the identified position(s) maybe used to control a display to generate a representation of the identified position at the display, e.g. by controlling signals provided at an output interface.

In some examples, the method 700 comprises a step 750 of obtaining indication data, e.g. at an input interface. The indication data is responsive to a change in one or more dielectric properties of elements within the crossing electric field and/or a property of the interventional device and/or electrodes.

The present disclosure recognizes that such indication data indicates whether the accuracy of the mapping function has been affected. In particular, changes in the dielectric properties of the crossing electric fields (and/or changes to the interventional device or electrode(s)) result in the mapping function no longer accurately mapping the electrical response(s) of the electrode(s) to the corresponding position(s) in the multidimensional co-ordinate system.

Thus, in some examples, if there is a detected change in the indication data (i.e. the indication data indicates a change beyond some predetermined threshold), then the method 600 of obtaining a new mapping function may be repeated. In other words, the mapping function may be regenerated. The detected change may be detected in a step 760.

Figure 7 also illustrates a computer-implemented method 70 of generating an anatomical model of an anatomical cavity.

The computer-implemented method 70 comprises iteratively identifying the position of one or more electrodes within an anatomical cavity by performing the method 700.

The computer-implemented method 70 also comprises a step 75 of generating an anatomical model of the anatomical cavity by processing the identified positions of the one or more electrodes. Step 75 may receive the positions from the output step 740 of method 700.

Methods for generating an anatomical cavity by processing identified positions have been previously described, e.g. with reference to at least Figures 3 and 4.

Step 75 may be repeated if, for instance, the indication data indicates a change, as this may indicate a change in the structure of the anatomical model.

A visual representation of the anatomical model may be provided, for example, at a user interface. Thus, step 75 may comprise controlling a user interface to provide a visual representation of the anatomical model.

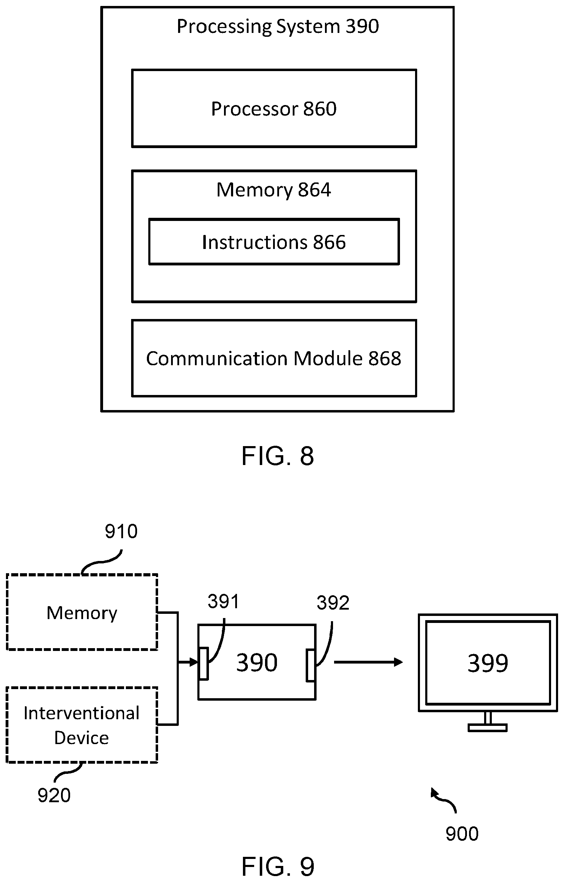

Figure 8 is a schematic diagram of a processing system 390, according to embodiments of the present disclosure. The processing system 390 is configured to carry out a method according to an embodiment, such as the method 600 described with reference to Figure 6.

The processing system 390 is therefore configured to generate a mapping function. The processing system 390 may also be configured to perform a dielectric imaging process, such as those previously described with reference to Figures 3 and 4.

As shown, the processing system 390 may include a (data) processor 860, a memory 864, and a communication module 868. These elements may be in direct or indirect communication with each other, for example via one or more buses.

The processor 860 may include a central processing unit (CPU), a digital signal processor (DSP), an ASIC, a controller, an FPGA, another hardware device, a firmware device, or any combination thereof configured to perform the operations described herein. The processor 860 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 864 may include a cache memory (e.g., a cache memory of the processor 860), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 864 includes a non-transitory computer-readable medium. The non-transitory computer-readable medium may store instructions. For example, the memory 864, or non-transitory computer-readable medium may have program code recorded thereon, the program code including instructions for causing the processing system 390, or one or more components of the processing system 390, particularly the processor 860, to perform the operations described herein. For example, the processing system 390 can execute operations of the method 600 and/or a method for performing a dielectric imaging process (as described with reference to Figures 3 and 4).

Instructions 866 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 864, with the code recorded thereon, may be referred to as a computer program product.

The communication module 868 can include any electronic circuitry and/or logic circuitry to facilitate direct or indirect communication of data between the processing system 390, the penetration device and/or the user interface (or other further device). In that regard, the communication module 868 can be an input/output (I/O) device. In some instances, the communication module 868 facilitates direct or indirect communication between various elements of the processing system 390 and/or the processing arrangement (Figure 9).

Figure 9 illustrates a processing arrangement 900 according to an embodiment of the invention, which illustrates some optional elements. The processing arrangement 900 comprises a processing system 390, which may be embodied as previously described.

The processing arrangement may further comprise an output display 399. The processing system may be configured to control the output display to display a visual representation of: the determined position of the electrode(s) and/or the interventional device; and/or the anatomical model, which are generated using the mapping function generated by the processing system.

This control may be performed using an output interface 392 of the processing system 390. The visual representation of the determined position(s) may be positioned with respect to the anatomical model, e.g. to indicate a relative position of the within the anatomical cavity represented by the anatomical model.

The processing system 390 may be adapted to obtain the responses of the one or more electrodes of the interventional device from a memory 910 and/or the interventional device 335, e.g. at an input interface 391. The processing arrangement 900 may comprise the memory and/or the interventional device 335 as appropriate.

The memory 910 may also provide the first machine-learning algorithm (or the plurality of first machine learning algorithms) and/or the initial mapping function (as appropriate). It will be appreciated that these could be obtained from another source, such as a user interface or an external server, e.g. contactable over a communication channel such as internet or a LAN connection).

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

## Claims

1. A computer-implemented method (600) for generating a mapping function (500, 635) that maps a response of an electrode (331, 332, 333) to crossing electric fields within a subject (190, 305), to a position in a multidimensional co-ordinate space, the computer-implemented method comprising:
obtaining (610) electrical responses of two or more electrodes mounted on an interventional device (335) to the crossing electric fields induced within the subject;
processing (620) the obtained electrical responses using a first machine-learning algorithm to obtain one or more values for a respective one or more parameters of the mapping function; and
defining (630) the mapping function based on the obtained one or more values for the respective one or more parameters of the mapping function.

2. The computer-implemented method (600) of claim 1, wherein the first machine-learning algorithm is specific to the type of the interventional device (335).

3. The computer-implemented method (600) of any of claims 1 or 2, wherein the method further comprises a step (640) of obtaining an initial mapping function (645), and the step (630) of defining the mapping function comprises defining one or more parameters of the initial mapping function based on the obtained one or more values.

4. The computer-implemented method (600) of claim 3, wherein the initial mapping function (645) is specific to the type of the interventional device (335) and/or the obtained electrical responses.

5. The computer-implemented method (600) of any of claims 3 or 4, wherein the first machine-learning algorithm is dependent upon the initial mapping function (645).

6. The computer-implemented method (600) of any of claims 1 to 5, further comprising a step (650) of obtaining the first machine-learning algorithm by processing the obtained electrical responses using a second machine-learning algorithm to identify one of a plurality of potential first machine-learning algorithms to use as the first-machine learning algorithm.

7. The computer-implemented method (600) of claim 6, wherein the step (650) of obtaining the first machine-learning algorithm comprises:
processing (651) the obtained electrical responses using the second machine-learning algorithm to identify a type of the interventional device; and
identifying (652) which potential first machine-learning algorithm to use as the first machine-learning algorithm based on the identified type of the interventional device.

8. The computer-implemented method (600) of any of claims 1 to 7, wherein the step (610) of obtaining electrical responses comprises obtaining at least some of the electrical responses from two or more electrodes (331, 332, 333) mounted on an interventional device (335) positioned within the subject (190, 305.

9. The computer-implemented method (600) of any of claims 1 to 8, wherein the electrical responses are electrical responses of the one or more electrodes within a predetermined part of an anatomical cycle of the subject.

10. The computer-implemented method (600) of claim 9, wherein the first machine-learning algorithm is dependent upon the predetermined part of the anatomical cycle of the subject.

11. A computer-implemented method (700) of identifying the position, in a multidimensional co-ordinate space, of an electrode (331, 332, 333) within a subject (190, 305), the computer-implemented method comprising:
obtaining (710) a mapping function (500, 635) generated using a method (600) according to one of claims 1 to 10;
obtaining (720) the electrical response of an electrode to crossing electric fields within the subject; and
determining (730) a position, in a multidimensional co-ordinate space, of the electrode by processing the electrical response of the electrode using the mapping function.

12. The computer-implemented method (700) of claim 11, further comprising:
obtaining (750) indication data responsive to a change in one or more dielectric properties of elements within the crossing electric field and/or a property of the interventional device and/or electrodes; and
in response to the indication data indicating a change, regenerating a mapping function by performing the method (600) of any of claims 1 to 10.

13. A computer-implemented method (70) of generating an anatomical model of an anatomical cavity, the computer-implemented method comprising:
iteratively identifying the position of one or more electrodes within an anatomical cavity by performing the method (700) of claim 11 or 12; and
generating (75) an anatomical model of the anatomical cavity by processing the identified positions of the one or more electrodes.

14. A computer program product comprising computer program code means which, when executed on a computing device having a processing system, cause the processing system to perform all of the steps of the method (600, 700) according to any of claims 1 to 13.

15. A processing system (390) for generating a mapping function (500,635) that maps a response of an electrode (331, 332, 333) to crossing electric fields within a subject (190, 305), to a position in a multidimensional co-ordinate space, the processing system being configured to:
obtain (610), at an input interface (391), electrical responses of two or more electrodes mounted on an interventional device (335) to the crossing electric fields induced within the subject;
process (620) the obtained electrical responses using a first machine-learning algorithm to obtain one or more values for a respective one or more parameters of the mapping function; and
define (630) the mapping function based on the obtained one or more values for the respective one or more parameters of the mapping function.
